(19) Europäisches Patentamt
European Patent Office
Office européen des brevets

(11) Veröffentlichungsnummer : **0 071 060**
**B1**

(12) # EUROPÄISCHE PATENTSCHRIFT

(45) Veröffentlichungstag der Patentschrift : **10.09.86**

(51) Int. Cl.⁴ : **C 07 D 403/10**, A 61 K 31/50

(21) Anmeldenummer : **82106111.6**

(22) Anmeldetag : **08.07.82**

(54) **6-(4-(Omega-(1-imidazolyl)-alkyl)-phenyl)-3-oxo-2,3,4,5-tetrahydro-pyridazine und deren Säureadditionssalze, Verfahren zu ihrer Herstellung und diese enthaltende pharmazeutische Präparate.**

(30) Priorität : **31.07.81 DE 3130251**

(43) Veröffentlichungstag der Anmeldung : **09.02.83 Patentblatt 83/06**

(45) Bekanntmachung des Hinweises auf die Patenterteilung : **10.09.86 Patentblatt 86/37**

(84) Benannte Vertragsstaaten : **AT BE CH FR GB IT LI LU NL SE**

(56) Entgegenhaltungen :
**DE-A- 2 151 216**
**GB-A- 1 383 906**
**I. Med. Chem. 1974, 17(3), 273-86 Curr. Sci. 1977, 46(23), 801-03**

(73) Patentinhaber : **A. Nattermann & Cie. GmbH**
**Nattermannallee 1**
**D-5000 Köln 30 (DE)**

(72) Erfinder : **Lautenschläger, Hans-Heiner, Dr.**
**Neusser Gasse 50**
**D-5024 Pulheim-Stommeln (DE)**
Erfinder : **Hilboll, Gerd, Dr.**
**Dehmelstrasse 36**
**D-5000 Köln 30 (DE)**
Erfinder : **Friehe, Hugo, Dr.**
**Am Burgfeld 94a**
**D-5042 Erftstadt-Lechenich (DE)**
Erfinder : **Löhr, Josef Peter, Dr.**
**Henkenheide 55**
**D-4010 Hilden (DE)**

(74) Vertreter : **Redies, Bernd, Dr. rer. nat.**
**COHAUSZ & FLORACK Patentanwaltsbüro Schumannstrasse 97 Postfach 14 01 47**
**D-4000 Düsseldorf 1 (DE)**

**EP 0 071 060 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 6-{4-[ω-(1-Imidazolyl)-alkyl]-phenyl}-3-oxo-2,3,4,5-tetrahydro-pyridazine, deren Säureadditionssalze, Verfahren zu ihrer Herstellung sowie ihre Verwendung als Wirkstoffe in Arzneimitteln, insbesondere zur Behandlung von entzündlichen und thromboembolischen Erkrankungen. Die erfindungsgemäßen 6-{4-[ω-(1-Imidazolyl)-alkyl]-phenyl}-3-oxo-2,3,4,5-tetrahydro-pyridazine entsprechen der allgemeinen Formel I

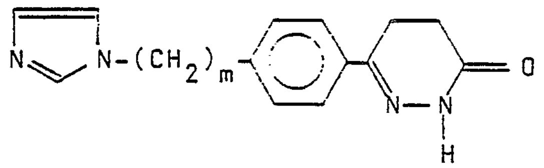

(I)

worin m eine ganze Zahl von 1-12, insbesondere 1-5, ist ; umfaßt sind auch die Säureadditionssalze der allgemeinen Formel I. Säureadditionssalze sind insbesondere pharmazeutisch verwendbare, untoxische Säureadditionssalze mit anorganischen Säuren, z. B. Chlorwasserstoffsäure, Bromwasserstoffsäure, Schwefelsäure oder Phosphorsäure, oder mit organischen Säuren, wie entsprechende Carbonsäuren, z. B. Essigsäure, Propionsäure, Oxalsäure, Malonsäure, Glykolsäure, Bernsteinsäure, Maleinsäure, Fumarsäure, Äpfelsäure, Weinsäure, Citronensäure, Benzoesäure oder Zimtsäure.

In der GB-PS 1 383 906 sind 6-Phenyl-4,5-dihydro-3(2H)-pyridazinone mit verschiedenen Substituenten in verschiedenen Stellungen des Phenylrestes als antihypertensive Wirkstoffe beschrieben. In der DE-OS 2 151 216 ist ebenfalls ein p-substituiertes 6-Phenyl-4,5-dihydro-3(2H)-pyridazinon als Wirkstoff ohne nähere Angaben beschrieben. Auch aus J. Med. Chem. 1974, 17(3), 273-286 und Curr. Sci. 1977, 46(23), 801-803 geht hervor, daß die 6-Phenyl-4,5-dihydro-3(2H)-pyridazinone allgemein eine antihypertensive Wirksamkeit verschiedener Stärke haben.

Die Verbindungen der Erfindung weisen zusätzlich zu der blutdruckregulierenden Wirksamkeit wertvolle Eigenschaften auf. Sie zeichnen sich einerseits durch eine starke Beeinflussung des Arachidonsäure-Metabolismus aus, andererseits zeigen sie eine antagonistische Wirkung bezüglich einiger durch PAF (Platelet Activating Factor) geregelter physiologischer Vorgänge. Die erfindungsgemäßen Verbindungen haben daher bzw. darüber hinaus eine starke antithrombotische sowie antiatherosklerotische und antirheumatische Aktivität. Daneben zeigen die Verbindungen der allgemeinen Formel I eine günstige Beeinflussung asthmatischer Beschwerden. Sie lassen sich insbesondere zur Behandlung von entzündlichen, atherosklerotischen bzw. thromboembolischen Erkrankungen, insbesondere beim Menschen, nutzen.

Die Darstellung der erfindungsgemäßen Substanzen erfolgt durch Umsetzung von 4-{4-[ω-(1-Imidazolyl)-alkyl]-phenyl}-4-oxo-buttersäuren oder deren Estern der allgemeinen Formel II mit Hydrazin, dessen Hydrat oder Salzen, wie Hydrochlorid oder Hydrosulfat, in wässrigen, wässrig-alkoholischen, alkoholischen Medien oder in indifferenten organischen Lösungsmitteln, wie z. B. Toluol bzw. deren Mischungen mit Wasser oder Alkohol bei Temperaturen von 0-150 °C, vorzugsweise in Ethanol oder Wasser. Die Reaktion kann gegebenenfalls durch Säuren, die in Form ihrer Hydraziniumsalze eingesetzt werden können, oder durch Basen, wie z. B. Erdalkalioxide, katalysiert werden.

Die Umsetzung wird durch folgende Reaktionsgleichung veranschaulicht :

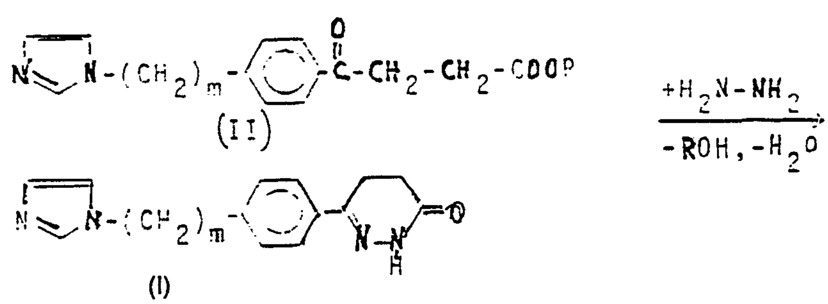


(I)

Als Ausgangsverbindungen der allgemeinen Formel II kommen insbesondere in Frage :
4-[4-(1-Imidazolylmethyl)-phenyl]-4-oxo-buttersäure sowie deren $C_{1-6}$-Alkylester ;
4-{4-[2-(1-Imidazolyl)-ethyl]-phenyl}-4-oxo-buttersäure sowie deren $C_{1-6}$-Alkylester ;
4-{4-[3-(1-Imidazolyl)-propyl]-phenyl}-4-oxo-buttersäure sowie deren $C_{1-6}$-Alkylester ;
4-{4-[4-(1-Imidazolyl)-butyl]-phenyl}-4-oxo-buttersäure sowie deren $C_{1-6}$-Alkylester ;
4-{4-[5-(1-Imidazolyl)-pentyl]-phenyl}-4-oxo-buttersäure sowie deren $C_{1-6}$-Alkylester ;
4-{4-[6-(1-Imidazolyl)-hexyl]-phenyl}-4-oxo-buttersäure sowie deren $C_{1-6}$-Alkylester ;
4-{4-[7-(1-Imidazolyl)-heptyl]-phenyl}-4-oxo-buttersaüre sowie deren $C_{1-6}$-Alkylester ;

4-{4-[8-(1-Imidazolyl)-octyl]-phenyl}-4-oxo-buttersäure sowie deren $C_{1-6}$-Alkylester ;
4-{4-[9-(1-Imidazolyl)-nonyl]-phenyl}-4-oxo-buttersäure sowie deren $C_{1-6}$-Alkylester ;
4-{4-[10-(1-Imidazolyl)-decyl]-phenyl}-4-oxo-buttersäure sowie deren $C_{1-6}$-Alkylester ;
4-{4-[11-(1-Imidazolyl)-undecyl]-phenyl}-4-oxo-buttersäure sowie deren $C_{1-6}$-Alkylester ;
4-{4-[12-(1-Imidazolyl)-dodecyl]-phenyl}-4-oxo-buttersäure sowie deren $C_{1-6}$-Alkylester ;

Die Darstellung der Ausgangsverbindungen der allgemeinen Formel II erfolgt nach an sich bekannten Verfahren :

1-(ω-Phenylalkyl)-imidazole werden durch Alkylierung von Imidazol mit dem entsprechenden ω-Halogenalkylbenzol gegebenenfalls unter Zusatz eines organischen Lösungsmittels, wie z. B. Dimethylformamid, unter möglicher Verwendung einer Hilfsbase, wie z. B. Natriumhydrid, hergestellt (DT-OS 2 933 649). Die 1-(ω-Phenylalkyl)-imidazole werden nach den dem Fachmann geläufigen Verfahren (Houben-Weyl, Methoden der organischen Chemie, Bd. 7/2a, S. 257 ff) mit Bernsteinsäurealkylesterchlorid unter Zusatz eines organischen Lösungsmittels, wie z. B. 1,2-Dichlorethan, Nitrobenzol, Schwefelkohlenstoff, unter Verwendung eines Friedel-Crafts-Katalysators, wie z. B. Aluminiumchlorid, zu den 4-{4-[ω-(1-Imidazolyl)-alkyl]-phenyl}-4-oxo-buttersäurealkylestern umgesetzt.

Die Säureadditionssalze von Verbindungen der allgemeinen Formel I mit anorganischen oder organischen Säuren lassen sich durch Mischen der zugrundeliegenden Imidazolylverbindungen mit den entsprechenden Säuren in wässrigen, wässrigorganischen (z. B. Alkohol-Wasser) oder organischen Medien, wie z. B. Alkoholen, Alkohol-Ether-Mischungen oder Ether-Petrolether-Mischungen bei Temperaturen zwischen 0 und 100 °C herstellen.

Die Erfindung betrifft ebenfalls pharmazeutische Präparate, welche Verbindungen der allgemeinen Formel I oder pharmazeutisch verwendbare Säureadditionssalze dieser Verbindungen enthalten. Bei den erfindungsgemäßen pharmazeutischen Präparaten handelt es sich um solche zur enteralen, wie oralen oder rektalen sowie parenteralen Verabreichung, welche die pharmakologischen Wirkstoffe allein oder zusammen mit einem üblichen, pharmazeutisch anwendbaren Trägermaterial enthalten.

Vorteilhafterweise liegt die pharmazeutische Zubereitung des Wirkstoffes in Formen von Einzeldosen vor, die auf die gewünschte Verabreichung abgestimmt sind, wie z. B. Tabletten, Dragees, Kapseln, Suppositorien, Granulate, Lösungen, Emulsionen oder Suspensionen. Die Dosierung der Verbindung liegt üblicherweise zwischen 1-500 mg pro Dosis, vorzugsweise zwischen 5-150 mg je Dosis und kann ein- oder mehrmals, bevorzugt zwei- bis dreimal täglich, verabreicht werden. Die Herstellung der erfindungsgemäßen Verbindung wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert. Die IR-Spektren wurden mit dem Gerät Perkin Elmer 257 und die Massenspektren mit dem Gerät Varian MAT-311-A (70 eV) aufgenommen.

## Beispiel 1

6-[4-(1-Imidazolylmethyl)-phenyl]-3-oxo-2,3,4,5-tetrahydropyridazin.

Eine Mischung aus 7,7 g 4-[4-(1-Imidazolylmethyl)-phenyl]-4-oxo-buttersäuremethylester, 0,2 g Bariumoxid, 1,4 g Hydrazinhydrat und 30 ml Ethanol wird 10 Minuten bei 0 °C, anschließend 2 Stunden bei Raumtemperatur gerührt und danach 1 Stunde unter Rückfluß erhitzt. Die Reaktionslösung wird mit Wasser verdünnt, mit Chloroform extrahiert und der Extrakt mit Wasser gewaschen und über $Na_2SO_4$ getrocknet. Das Lösungsmittel wird abgezogen und der zurückbleibende Rückstand durch Ausrühren mit Ether gereinigt.

Ausbeute : 2,4 g, Fp. 225 °C
IR (in KBr) : 1 675 $cm^{-1}$
MS [m/e] : 254 ($M^+$, 40 %), 187 (100 %), 116 (21 %)

## Beispiel 2

6-{4-[2-(1-Imidazolyl)-ethyl]-phenyl}-3-oxo-2,3,4,5-tetrahydro-pyridazin.

Eine Mischung aus 7,5 g 4-{4-[2-(1-Imidazolyl)-ethyl]-phenyl}-4-oxo-buttersäuremethylester, 0,2 g Bariumoxid, 1,3 g Hydrazinhydrat und 30 ml Ethanol wird 10 Minuten bei 0 °C, anschließend 2 Stunden bei Raumtemperatur gerührt und danach 1 Stunde unter Rückfluß erhitzt. Die Reaktionsmischung wird mit Wasser verdünnt, mit Chloroform extrahiert und der Extrakt mit Wasser gewaschen und über $Na_2SO_4$ getrocknet. Das Lösungsmittel wird abgezogen und der Rückstand durch Säulenchromatographie gereinigt (Kieselgel/Chloroform/Methanol).

Ausbeute : 3,6 g, Fp. 145 °C
IR (in KBr) : 1 660 $cm^{-1}$
MS [m/e] : 268 ($M^+$, 100 %), 187 (67 %), 116 (17 %), 81 (22 %)

## Beispiel 3

6-{4-[3-(1-Imidazolyl)-propyl]-phenyl}-3-oxo-2,3,4,5-tetrahydro-pyridazin.

2 g 4-{4-[3-(1-Imidazolyl)-propyl]-phenyl}-4-oxo-buttersäure werden in 10 ml Wasser suspendiert,

0,68 g Hydrazinhydrat werden hinzugefügt und anschließend wird die Mischung 2 Stunden bei 90 °C gerührt. Nach dem Abkühlen wird mit Chloroform extrahiert, die Chloroform-Lösung mit Wasser gewaschen, über $Na_2SO_4$ getrocknet und zur Trockne eingeengt.

Ausbeute : 1,2 g, Fp. 136 °C

IR (in KBr) : 1 675 $cm^{-1}$

MS [m/e] : 282 ($M^+$, 100 %), 266 (0,7 %), 253 (10 %), 240 (13 %), 212 (11 %), 186 (10 %)

Beispiel 4

6-{4-[4-(1-Imidazolyl)-butyl]-phenyl}-3-oxo-2,3,4,5-tetrahydro-pyridazin.

a) Aus 4-{4-[4-(1-Imidazolyl)-butyl]-phenyl}-4-oxo-buttersäure-methylester

Eine Mischung aus 10 g Ester, 0,4 g Bariumoxid, 1,58 g Hydrazinhydrat und 30 ml Ethanol wird 10 Minuten bei 0 °C gerührt, anschließend 20 Stunden bei Raumtemperatur und noch 6 Stunden unter Rückfluß erhitzt. Nach dem Abkühlen wird das Lösungsmittel abgezogen, der Rückstand mit Wasser aufgenommen und mit Chloroform extrahiert. Die Chloroform-Phase wird mit Wasser gewaschen, über $Na_2SO_4$ getrocknet und eingeengt. Der Rückstand wird durch Säulenchromatographie gereinigt (Kieselgel/Chloroform/Methanol).

Ausbeute : 3,9 g, Fp. 131 °C

IR (in KBr) : 1 680 $cm^{-1}$

MS [m/e] : 296 ($M^+$, 100 %), 267 (15 %), 254 (16 %), 226 (18 %), 200 (10 %), 69 (35 %)

b) Aus 4-{4-[4-(1-Imidazolyl)-butyl]-phenyl}-4-oxo-buttersäure.

1,5 g Säure werden in 10 ml Wasser suspendiert. Nach Zugabe von 0,3 g Hydrazinhydrat wird 2 Stunden bei 90 °C gerührt. Nach dem Abkühlen wird mit Chloroform extrahiert, die Chloroform-Phase mit Wasser gewaschen, über $Na_2SO_4$ getrocknet und zur Trockne eingeengt.

Ausbeute : 1,25 g, Fp. 129-131 °C

Beispiel 5

6-{4-[5-(1-Imidazolyl)-pentyl]-phenyl}-3-oxo-2,3,4,5-tetrahydro-pyridazin.

a) Aus 4-{4-[5-(1-Imidazolyl)-pentyl]-phenyl}-4-oxo-buttersäuremethylester.

Eine Mischung aus 10,5 g Ester, 0,4 g Bariumoxid, 1,58 g Hydrazinhydrat und 30 ml Ethanol wird 10 Minuten bei 0 °C gerührt, anschließend 18 Stunden bei Raumtemperatur und noch 4 Stunden unter Rückfluß erhitzt. Nach dem Abkühlen wird das Lösungsmittel abgezogen, der Rückstand in Wasser aufgenommen und mit Chloroform extrahiert. Die Chloroformphase wird mit Wasser gewaschen, über $Na_2SO_4$ getrocknet und eingeengt. Der Rückstand wird durch Säulenchromatographie gereinigt (Kieselgel/Chloroform/Methanol).

Ausbeute : 2,3 g, Fp. 124 °C

IR (in KBr) : 1680 $cm^{-1}$

MS [m/e] : 310 ($M^+$, 48 %), 294 (3 %), 281 (16 %), 268 (10 %), 240 (57 %), 214 (23 %) 69 (100 %)

b) Aus 4-{4-[5-(1-Imidazolyl)-pentyl]-phenyl}-4-oxo-buttersäure.

3,14 g Säure werden in 10 ml Wasser suspendiert. Nach Zugabe von 0,6 g Hydrazinhydrat wird 2 Stunden bei 90 °C gerührt. Nach dem Abkühlen wird abgesaugt, der Rückstand mit Wasser gewaschen und getrocknet.

Ausbeute : 2,56, Fp. 124-126 °C

Darstellung der Ausgangsverbindung

4-{4-[4-(1-Imidazolyl)-butyl]-phenyl}-4-oxo-buttersäuremethylester.

Zu einer Suspension von 59,3 g Aluminiumchlorid in 200 ml 1,2-Dichlorethan werden unter Eiskühlung 20,3 g Bernsteinsäuremethylesterchlorid und anschließend eine Lösung von 27 g 1-(4-Phenylbutyl)-imidazol in 100 ml 1,2-Dichlorethan zugetropft. Danach wird 3 Stunden bei 85 °C gerührt. Nach dem Abkühlen wird die Reaktionsmischung in eine Mischung aus 165,7 g Ethylendiamintetraessigsäure und 600 g Eis eingerührt und durch Zugabe von verdünnter Natronlauge auf ca. pH 8 gebracht. Die Phasen werden getrennt, die organische Phase über $Na_2SO_4$ getrocknet und eingeengt. Der Rückstand wird durch Säulenchromatographie gereinigt (Kieselgel/Chloroform).

Ausbeute : 32 g, Fp. 53-54 °C

IR (in KBr) : 1 730, 1 680 $cm^{-1}$

MS [m/e] : 314 ($M^+$, 25 %), 283 (14 %), 255 (21 %), 227 (100 %), 200 (15 %), 131 (16 %), 96 (66 %)

4-{4-[4-(1-Imidazolyl)-butyl]-phenyl}-4-oxo-buttersäure.

15 g 4-{4-[4-(1-Imidazolyl)-butyl]-phenyl}-4-oxo-buttersäuremethylester werden in 180 ml Methanol gelöst und 2,3 g Natriumhydroxid, gelöst in wenig Methanol, hinzugefügt. Die Mischung wird 24 Stunden bei Raumtemperatur gerührt, das Lösungsmittel abgezogen und der Rückstand in Wasser aufgenommen. Die Lösung wird mehrfach mit Chloroform extrahiert, die Chloroformphase verworfen. Die wässrige Lösung wird mit verdünnter Salzsäure auf ca. pH 6 eingestellt, der ausgefallene Feststoff abgesaugt, mit

wenig Wasser gewaschen und getrocknet.

Ausbeute : 8,8 g, Fp. 165-166 °C

IR (in KBr) : 1 710, 1 680 $cm^{-1}$

MS [m/e] : 299 ($M^+$ -1, 0,5 %), 282 (4 %), 256 (13 %), 227 (35 %), 200 (100 %), 131 (18 %), 96 (28 %), 82 (28 %), 69 (33 %)

Analog den Beispielen 1-5 werden hergestellt :

6. 6-{4-[6-(1-Imidazolyl)-hexyl]-phenyl}-3-oxo-2,3,4,5-tetrahydro-pyridazin
7. 6-{4-[7-(1-Imidazolyl)-heptyl]-phenyl}-3-oxo-2,3,4,5-tetrahydro-pyridazin
8. 6-{4-[8-(1-Imidazolyl)-octyl]-phenyl}-3-oxo-2,3,4,5-tetrahydro-pyridazin
9. 6-{4-[9-(1-Imidazolyl)-nonyl]-phenyl}-3-oxo-2,3,4,5-tetrahydro-pyridazin
10. 6-{4-[10-(1-Imidazolyl)-decyl]-phenyl}-3-oxo-2,3,4,5-tetrahydro-pyridazin
11. 6-{4-[11-(1-Imidazolyl)-undecyl]-phenyl}-3-oxo-2,3,4,5-tetrahydro-pyridazin
12. 6-{4-[12-(1-Imidazolyl)-dodecyl]-phenyl}-3-oxo-2,3,4,5-tetrahydro-pyridazin

## Beispiel 13

6-{4-[5-(1-Imidazolyl)-pentyl]-phenyl}-3-oxo-2,3,4,5-tetrahydro-pyridazin-Fumarsäure-Salz.

Eine Mischung aus 1,5 g 6-{4-[5-(1-Imidazolyl)-pentyl]-phenyl}-3-oxo-2,3,4,5-tetrahydro-pyridazin und 0,55 g Fumarsäure wird in 30 ml Ethanol ca. 30 Minuten unter Rückfluß erhitzt, bis sich eine klare Lösung gebildet hat. Beim Abkühlen kristallisiert das Salz aus, das abgesaugt und getrocknet wird.

Ausbeute : 1,73 g, Fp. 138 °C

IR (in KBr) : 1 680 $cm^{-1}$

Analog Beispiel 13 lassen sich z. B. Oxalate, Succinate und Malonate sowie anorganische Salze, wie Hydrochloride und Hydrosulfate herstellen.

**Patentansprüche** (für die Vertragsstaaten : BE, CH, FR, GB, IT, LI, LU, NL, SE)

1. 6-{4-[ω-(1-Imidazolyl)-alkyl]-phenyl}-3-oxo-2,3,4,5, tetrahydropyridazine der allgemeinen Formel I

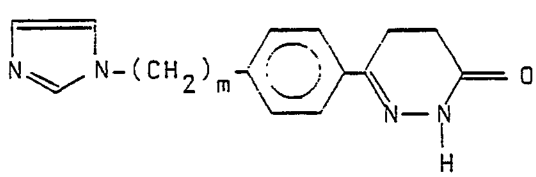

(I)

in der m eine ganze Zahl von 1-12 bedeutet, sowie deren Säureadditionssalze mit anorganischen oder organischen Säuren.

2. 6-[4-(1-Imidazolylmethyl)-phenyl]-3-oxo-2,3,4,5-tetrahydro-pyridazin und dessen pharmazeutisch verträgliche Säureadditionssalze.

3. 6-{4-[2-(1-Imidazolyl)-ethyl]-phenyl}-3-oxo-2,3,4,5-tetrahydro-pyridazin und dessen pharmazeutisch verträgliche Säureadditionssalze.

4. 6-{4-[3-(1-Imidazolyl)-propyl]-phenyl}-3-oxo-2,3,4,5-tetrahydro-pyridazin und dessen pharmazeutisch verträgliche Säureadditionssalze.

5. 6-{4-[4-(1-Imidazolyl)-butyl]-phenyl}-3-oxo-2,3,4,5-tetrahydro-pyridazin und dessen pharmazeutisch verträgliche Säureadditionssalze.

6. 6-{4-[5-(1-Imidazolyl)-pentyl]-phenyl}-3-oxo-2,3,4,5-tetrahydro-pyridazin und dessen pharmazeutisch verträgliche Säureadditionssalze.

7. 6-{4-[6-(1-Imidazolyl)-hexyl]-phenyl}-3-oxo-2,3,4,5-tetrahydro-pyridazin und dessen pharmazeutisch verträgliche Säureadditionssalze.

8. 6-{4-[7-(1-Imidazolyl)-heptyl]-phenyl}-3-oxo-2,3,4,5-tetrahydro-pyridazin und dessen pharmazeutisch verträgliche Säureadditionssalze.

9. 6-{4-[8-(1-Imidazolyl)-octyl]-phenyl}-3-oxo-2,3,4,5-tetrahydro-pyridazin un dessen pharmazeutisch verträgliche Säureadditionssalze.

10. 6-{4-[9-(1-Imidazolyl)-nonyl]-phenyl}-3-oxo-2,3,4,5-tetrahydro-pyridazin und dessen pharmazeutisch verträgliche Säureadditionssalze.

11. 6-{4-[10-(1-Imidazolyl)-decyl]-phenyl}-3-oxo-2,3,4,5-tetrahydro-pyridazin und dessen pharmazeutisch verträgliche Säureadditionssalze.

12. 6-{4-[11-(1-Imidazolyl)-undecyl]-phenyl}-3-oxo-2,3,4,5-tetrahydro-pyridazin und dessen pharmazeutisch verträgliche Säureadditionssalze.

13. 6-{4-[12-(1-Imidazolyl)-dodecyl]-phenyl}-3-oxo-2,3,4,5-tetrahydro-pyridazin und dessen pharmazeutisch verträgliche Säureadditionssalze.

14. Verfahren zur Herstellung der Verbindungen gemäß den Ansprüchen 1-13, dadurch gekennzeichnet, daß man eine 4-{4-[ω-(1-Imidazolyl)-alkyl]-phenyl}-4-oxo-buttersäure oder deren Ester der allgemeinen Formel II

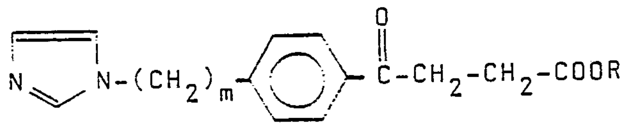

(II)

wobei m die in der allgemeinen Formel I angegebene Bedeutung hat und R Wasserstoff oder $C_{1-6}$-Alkyl bedeutet, mit Hydrazin, dessen Hydrat oder einem Hydrazinsalz in wäßrigen, wäßrig-alkoholischen, alkoholischen Medien oder indifferenten, organischen Lösungsmitteln oder deren Mischungen mit Wasser oder Alkohol bei einer Temperatur im Bereich von 0-150 °C, gegebenenfalls unter Zuhilfenahme eines der für Aminolysen und Kondensationsreaktionen üblichen Katalysatoren umsetzt.

15. Arzneimittelzubereitungen, dadurch gekennzeichnet, daß sie eine Verbindung oder ein pharmazeutisch verträgliches Salz dieser Verbindung gemäß den Ansprüchen 1-13 zusammen mit einem pharmazeutisch geeigneten Verdünnungsmittel oder Trägermaterial enthalten.

**Patentanspruch** (für den Vertragsstaat AT)

Verfahren zur Herstellung von 6-{4-[ω-(1-Imidazolyl)-alkyl]-phenyl}-3-oxo-2,3,4,5-tetrahydropyridazinen der allgemeinen Formel I

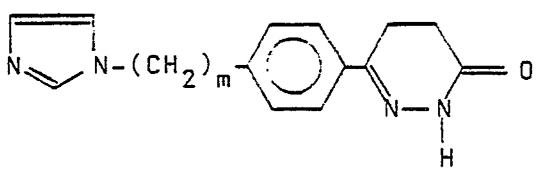

(I)

in der m eine ganze Zahl von 1-12 bedeutet, sowie deren Säureadditionssalzen mit anorganischen oder organischen Säuren, dadurch gekennzeichnet, daß man eine 4-{4-[ω-(1-Imidazolyl)-alkyl]-phenyl}-4-oxo-buttersäure oder deren Ester der allgemeinen Formel II

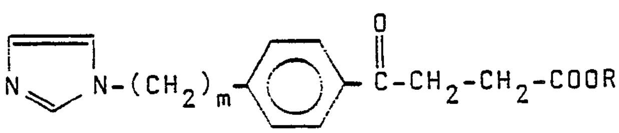

(II)

wobei m die in der allgemeinen Formel I angegebene Bedeutung hat und R Wasserstoff oder $C_{1-6}$-Alkyl bedeutet, mit Hydrazin, dessen Hydrat oder einem Hydrazinsalz in wäßrigen, wäßrig-alkoholischen, alkoholischen Medien oder indifferenten, organischen Lösungsmitteln oder deren Mischungen mit Wasser oder Alkohol bei einer Temperatur im Bereich von 0-150 °C, gegebenenfalls unter Zuhilfenahme eines der für Aminolysen und Kondensationsreaktionen üblichen Katalysatoren umsetzt.

**Claims** (for the Contracting states : BE, CH, FR, GB, IT, LI, LU, NL, SE)

1. 6-{4-[ω-(1-Imidazolyl)-alkyl]-phenyl}-3-oxo-2,3,4,5-tetrahydropyridazine of the general formula I

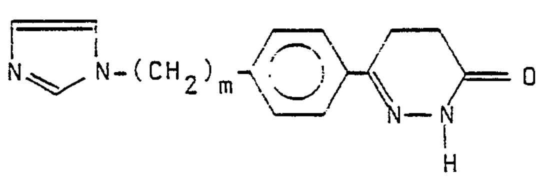

(I)

wherein m is a numeral of from 1 to 12, as well as the acid addition salts with inorganic or organic acids.

2. 6-[4-(1-Imidazolylmethyl)-phenyl]-3-oxo-2,3,4,5-tetrahydropyridazine and its pharmaceutically compatible salts.

3. 6-{4-[2-(Imidazolyl)-ethyl]-phenyl}-3-oxo-2,3,4,5-tetrahydropyridazine and its pharmaceutically compatible salts.

4. 6-{4-[3-(1-Imidazolyl)-propyl]-phenyl}-3-oxo-2,3,4,5-tetrahydropyridazine and its pharmaceutically compatible salts.

5. 6-{4-[4-(1-Imidazolyl)-butyl]-phenyl}-3-oxo-2,3,4,5-tetrahydropyridazine and its pharmaceutically compatible salts.

6. 6-{4-[5-(1-Imidazolyl)-pentyl]-phenyl}-3-oxo-2,3,4,5-tetrahydropyridazine and its pharmaceutically compatible acid addition salts.

7. 6-{4-[6-(1-Imidazolyl)-hexyl]-phenyl}-3-oxo-2,3,4,5-tetrahydropyridazine and its pharmaceutically compatible acid addition salts.

8. 6-{4-[7-(1-Imidazolyl)-heptyl]-phenyl}-3-oxo-2,3,4,5-tetrahydropyridazine and its pharmaceutically compatible acid addition salts.

9. 6-{4-[8-(1-Imidazolyl)-octyl]-phenyl}-3-oxo-2,3,4,5-tetrahydropyridazine and its pharmaceutically compatible acid addition salts.

10. 6-{4-[9-(1-Imidazolyl)-nonyl]-phenyl}-3-oxo-2,3,4,5-tetrahydropyridazine and its pharmaceutically compatible acid addition salts.

11. 6-{4-[10-(1-Imidazolyl)-decyl]-phenyl}-3-oxo-2,3,4,5-tetrahydropyridazine and its pharmaceutically compatible acid addition salts.

12. 6-{4-[11-(1-Imidazolyl)-undecyl]-phenyl}-3-oxo-2,3,4,5-tetrahydropyridazine and its pharmaceutically compatible acid addition salts.

13. 6-{4-[12-(1-Imidazolyl)-dodecyl]-phenyl}-3-oxo-2,3,4,5-tetrahydropyridazine and its pharmaceutically compatible acid addition salts.

14. Process for the preparation of compounds according to claims 1 to 13, characterised in that a 4-{4-[ω-(1-Imidazolyl)-alkyl]-phenyl}-4-oxo-butyric acid or an ester thereof having the general formula II

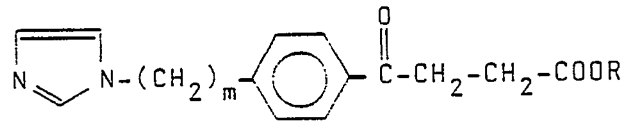

(II)

wherein m has the meaning as given in formula I and R is hydrogen or a $C_{1-6}$-alkyl group, is subjected to reaction with hydrazine, its hydrate or a salt of hydrazine, in an aqueous, aqueous-alcoholic or alcoholic reaction mixture or in an inert organic solvent or its mixtures with water or alcohol at a temperatur of from 0 to 150 °C, possibly in the presence of a catalyst usual for ammolytic acid condensation reactions.

15. Pharmaceutical preparations characterised in that they contain a compound or a pharmaceutically compatible acid addition salt thereof as claimed in claims 1 to 13 together with parmaceutically suitable diluents or carrier materials.

**Claim** (for the Contracting state AT)

Process for the production of 6-{4-[ω-(1-imidazolyl)-alkyl]-phenyl}-3-oxo-2,3,4,5-tetrahydropyridazines of the general formula I

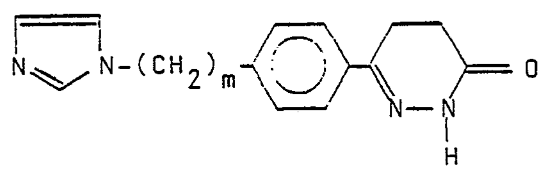

(I)

wherein m is a numeral of from 1 to 12, as well as the acid addition salts with inorganic or organic acids, characterised in that a 4-{4-[ω-(1-imidazolyl)-alkyl]-phenyl}-4-oxo-butyric acid or an ester thereof having the general formula II

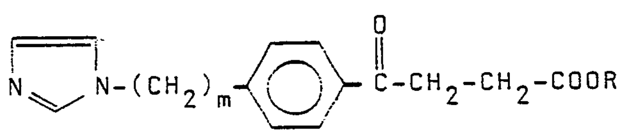

(II)

wherein m has the meaning as given in formula I and R is hydrogen or a $C_{1-6}$-alkyl group, is subjected to reaction with hydrazine, its hydrate or a salt of hydrazine, in an aqueous, aqueous-alcoholic or alcoholic reaction mixture or in an inert organic solvent or its mixtures with water or alcohol at a temperature of from 0 to 150 °C, possibly in the presence of a catalyst usual for ammolytic acid condensation reactions.

**Revendications** (pour les Etats contractants : BE, CH, FR, GB, IT, LI, LU, NL, SE)

1. 6-{4-[ω-(1-Imidazolyl)-alcoyl]-phényl}-3-oxo-2,3,4,5-tétrahydropyridazines de formule générale

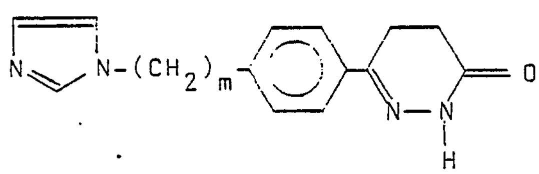

(I)

où m représente un nombre entier allant de 1 à 12, ainsi que leurs sels d'addition d'acides avec des acides inorganiques ou organiques.

2. 6-[4-(1-Imidazolylméthyl)-phényl]-3-oxo-2,3,4,5-tétrahydro-pyridazine et ses sels d'addition d'acides pharmaceutiquement acceptables.

3. 6-{4-[2-(1-Imidazolyl)-éthyl]-phényl}-3-oxo-2,3,4,5-tétrahydro-pyridazine et ses sels d'addition d'acides pharmaceutiquement acceptables.

4. 6-{4-[3-(1-Imidazolyl)-propyl]-phényl}-3-oxo-2,3,4,5-tétrahydro-pyridazine et ses sels d'addition d'acides pharmaceutiquement acceptables.

5. 6-{4-[4-(1-Imidazolyl)-butyl]-phényl}-3-oxo-2,3,4,5-tétrahydro-pyridazine et ses sels d'addition d'acides pharmaceutiquement acceptables.

6. 6-{4-[5-(1-Imidazolyl)-pentyl]-phényl}-3-oxo-2,3,4,5-tétrahydro-pyridazine et ses sels d'addition d'acides pharmaceutiquement acceptables.

7. 6-{4-[6-(1-Imidazolyl)-hexyl]-phényl}-3-oxo-2,3,4,5-tétrahydro-pyridazine et ses sels d'addition d'acides pharmaceutiquement acceptables.

8. 6-{4-[7-(1-Imidazolyl)-heptyl]-phényl}-3-oxo-2,3,4,5-tétrahydro-pyridazine et ses sels d'addition d'acides pharmaceutiquement acceptables.

9. 6-{4-[8-(1-Imidazolyl)-octyl]-phényl}-3-oxo-2,3,4,5-tétrahydro-pyridazine et ses sels d'addition d'acides pharmaceutiquement acceptables.

10. 6-{4-[9-(1-Imidazolyl)-nonyl]-phényl}-3-oxo-2,3,4,5-tétrahydro-pyridazine et ses sels d'addition d'acides pharmaceutiquement acceptables.

11. 6-{4-[10-(1-Imidazolyl)-décyl]-phényl}-3-oxo-2,3,4,5-tétrahydro-pyridazine et ses sels d'addition d'acides pharmaceutiquement acceptables.

12. 6-{4-[11-(1-Imidazolyl)-undécyl]-phényl}-3-oxo-2,3,4,5-tétrahydro-pyridazine et ses sels d'addition d'acides pharmaceutiquement acceptables.

13. 6-{4-[12-(1-Imidazolyl)-dodécyl]-phényl}-3-oxo-2,3,4,5-tétrahydro-pyridazine et ses sels d'addition d'acides pharmaceutiquement acceptables.

14. Procédé de préparation des composés selon les revendications 1-13 caractérisé en ce qu'on fait réagir un acide 4-{4-[ω-(1-imidazolyl)-alcoyl]-phényl}-4-oxo-butyrique ou ses esters de formule générale II

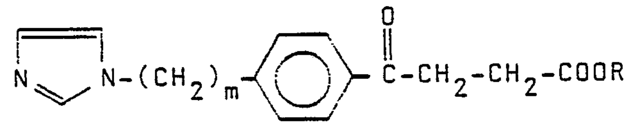

(II)

où m a la signification donnée dans la formule générale I et R représente un hydrogène ou un alcoyle en $C_1$ à $C_6$, avec de l'hydrazine, son hydrate ou un sel d'hydrazine dans les milieux aqueux, alcooliques aqueux, ou des solvants organiques indifférents ou leurs mélanges avec l'eau ou l'alcool à une température située dans un intervalle de 0-150 °C, éventuellement à l'aide d'un catalyseur habituel pour les aminolyses et les réactions de condensation.

15. Préparations médicamenteuses, caractérisées en ce qu'elles contiennent un composé ou un sel pharmaceutiquement acceptable de ce composé selon les revendications 1-13 avec un diluant ou support pharmaceutiquement approprié.

**Revendication** (pour l'Etat contractant AT)

Procédé de préparation de 6-{4-[ω-(1-imidazolyl)-alcoyl]-phényl}-3-oxo-2,3,4,5-tétrahydro-pyridazines de formule générale I

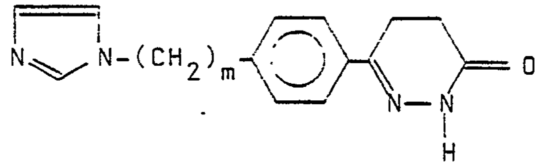

(I)

où m représente un nombre entier allant de 1 à 12, ainsi que de leurs sels d'addition d'acides avec des acides inorganiques ou organiques, caractérisé en ce qu'on fait réagir un acide 4-{4-[ω-(1-imidazolyl)-alcoyl]-phényl}-4-oxo-butyrique ou ses esters de formule générale II

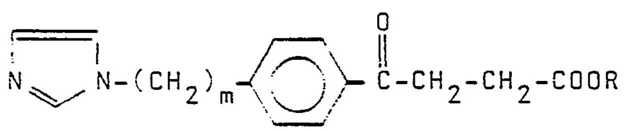

(II)

où m a la signification donnée dans la formule générale I et R représente un hydrogène ou un alcoyle en

$C_1$ à $C_6$, avec de l'hydrazine, son hydrate ou un sel d'hydrazine dans des milieux aqueux, alcooliques aqueux, alcooliques, ou des solvants organiques indifférents ou leurs mélanges avec l'eau ou l'alcool à une température située dans un intervalle allant de 0 à 150 °C, éventuellement à l'aide d'un catalyseur habituel pour les aminolyses et les réactions de condensation.